# EUROPEAN PATENT APPLICATION

(11) **EP 0 707 850 A1**
(43) Date of publication of application: **24.04.1996**
(21) Application number: 95306505.9
(22) Date of filing: 14.09.1995
(51) Int. Cl.: A61K 31/20

(54) **Use of polyunsaturated fatty acids for the manufacture of a medicament for the treatment of brest pain**

(30) Priority: 21.09.1994 GB 9419013; 13.02.1995 GB 9502742
(71) Applicant: SCOTIA HOLDINGS PLC, Guildford Surrey GU1 1BA (GB)
(72) Inventor: Horrobin, David Frederick, c/o Scotia Pharma. Ltd., Guildford, Surrey GU1 1BA (GB)
(74) Representative: Sturt, Clifford Mark

(57) **Abstract**

A method of treatment or prevention of, or a method of making a medicament for the treatment or prevention of, hormone replacement therapy-associated breast pain in peri- or post-menopausal women, wherein an n-6 or n-3 series essential fatty acid is used as an active of said medicament or in said therapy, optionally with a natural or synthetic replacement therapy oestrogen.

## Description

Hormone replacement therapy (HRT) using various natural and synthetic oestrogens is increasingly common in peri-menopausal and post-menopausal women. The main aims are to counteract the loss of natural oestrogen stimulation of the bones, the skin, and the genital tissues. The strongest reason for using HRT in most women is the prevention of osteoporosis. Through the complications of pathological fractures, osteoporosis kills more women than breast cancer.

One of the main problems of HRT is that many women develop breast pain because of oestrogen stimulation of the breast tissue. Depending on the dose and type of oestrogen used, the percentage of women taking HRT who experience breast pain may range from 30-50%. Breast pain is a major reason for women discontinuing HRT and so becoming deprived of the benefits.

Oestrogens can only produce a response in a tissue if that tissue contains proteins known as oestrogen receptors. The receptors bind oestrogen and transmit the oestrogen message to the nucleus of the cell. The oestrogen receptor is usually surrounded by a lipid annulus. Researchers have found that when the lipid environment is rich in saturated fats the oestrogen receptor increases its affinity for oestrogen and so is able to bind a greater proportion of the oestrogen available in the environment. Unsaturated fatty acids have the opposite effect, reducing the affinity of the receptor for oestrogen.

The concept has now been developed that HRT-associated breast pain may occur in women in whom the lipid environment of the oestrogen receptor is unduly rich in saturated fatty acids. This increases the affinity of the receptor for oestrogen and leads to an exaggerated response to a normal level of oestrogen. As a result of such an exaggerated response of breast tissue, pain results.

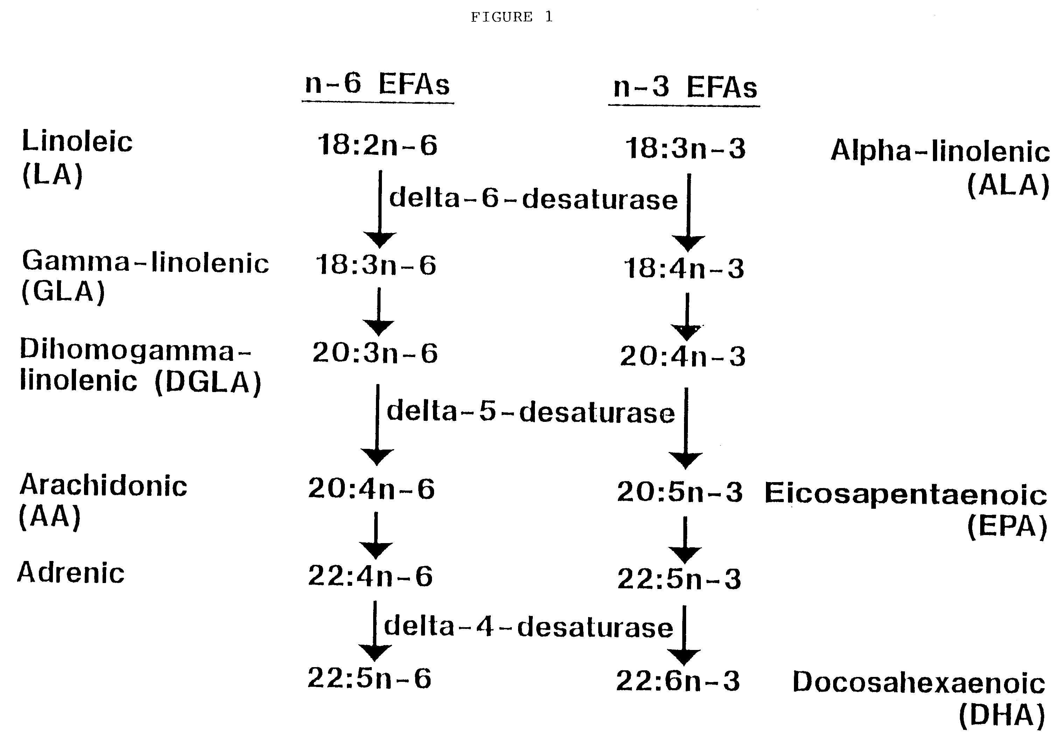

The concept has been tested by using gamma-linolenic acid (GLA), a highly unsaturated fatty acid with 3 double bonds. GLA is a member of the omega-6 family of essential fatty acids (EFAs) shown in figure 1. The related omega-3 EFAs also shown in figure 1 are also highly unsaturated.

Twenty women taking HRT who complained of painful breasts were given 320mg of GLA each day in soft gelatin capsules for a period of 3 months. Most of the women were taking either conjugated oestrogens (Premarin, 0.625mg, 1-3/day) or conjugated oestrogens for 16 days of each cycle with conjugated oestrogens and a progestin for 12 days (Prempak) or were using an oestradiol patch providing 25, 50 or 100 microg oestradiol every three days. At the end of this time the pain had completely disappeared in 9, was substantially reduced in a further 8, and remained a problem only in the remaining 3. In another study 20 women starting HRT were given their oestrogen in association with 320mg of GLA per day. Normally 8-12 of these women would have been expected to develop breast pain within 2-3 months but in fact only 2 developed pain which was only mild. Thus GLA is able to prevent and to treat HRT-associated breast pain.

GLA is very rapidly converted in the body to dihomogammalinolenic acid (DGLA) and DGLA will have the same therapeutic effect as GLA. Other EFAs shown in figure 1 have similar effects since they are highly unsaturated and if incorporated in the lipids surrounding the oestrogen receptor they will reduce its affinity for oestrogen.

The dose of GLA, DGLA or other fatty acid to be used may be from 1mg to 10g/day, preferably 50mg to 2g, and very preferably 100mg to 1g. The fatty acid may be provided in any form capable of being assimilated into the body such as free fatty acids, salts, mono-, di- or tri-glycerides, amides, alcohols, esters, phospholipids, cholesterol esters or any other appropriate form. The fatty acids may be used singly or in association with other essential fatty acids. They may be administered orally, enterally, parenterally, topically, via suppositories or pessaries or in any other appropriate form.

The fatty acids may be administered separately from the oestrogen in a different dosage form or may be formulated in either oral or topical, including vaginal, forms so that both the fatty acid and the oestrogen are delivered together.

The invention broadly is set out in the claims, to which reference should be made, but includes:-
1. Methods of treating or preventing HRT-associated breast pain by co-administering GLA or DGLA or other n-6 or n-3 EFA, and the replacement-therapy oestrogen, as a single formulation or separately.
2. Formulations of natural or synthetic oestrogens, and GLA or DGLA or other n-6 or n-3 EFA.
3. A method of making a medicament for use against HRT-associated breast pain, wherein natural or synthetic oestrogens and GLA or DGLA or other n-6 or n-3 EFA, or the EFA without the oestrogen where the medicament is for co-administration with the oestrogen, is or are used as the active(s) of said medicament.

In the light of the above, the skilled man will readily be able to apply the invention using per se conventional dosage forms, diluents or carriers, and the like. Particular examples however follow.

### Examples

The trials set out above are examples of the treatment of actual or potential HRT-associated breast pain.

The GLA or DGLA may be provided as free fatty acid, ester, glyceride, phospholipid or any other derivative which is known to those skilled in the art and which raise the levels of GLA and/or DGLA in the blood stream. Specific examples are:
1. Soft or hard gelatin capsules containing 20-500mg of GLA or DGLA in the form of a natural oil such as evening primrose, borage, blackcurrant or fungal oils or as a purified, semi-purified. natural or semi-synthetic triglyceride, to be taken in amounts which will deliver between 200 and 2000mg of GLA or DGLA per day and to be used in association with any appropriate natural or synthetic oestrogen alone or combined oestrogen/progestin therapy employed for the management of menopausal symptoms, or ovarian replacement or treatment or prevention of osteoporosis.
2. Other chemical forms such as free acids, esters or phospholipids, or other dosage forms such as tablets, emulsions, whips, creams, powders, microencapsulates for oral administration or pessaries or suppositories, or any other appropriate dosage form known to those skilled in the art which will deliver between 200 and 2000mg of GLA or DGLA per day.
3. Topical dosage forms such as creams, lotions, patches or other forms known to those skilled in the art which will provide 200 to 2000mg of GLA or DGLA per day.
4. Oral dosage forms in which the GLA or DGLA and the oestrogen or oestrogen/progestin are provided in the same tablet or capsule. For example a capsule containing 500 microg of conjugated oestrogens or 20 microg of oestradiol or an appropriate dose of any other oestrogen or progestin could be prepared which also contained 200 to 1000mg of GLA or DGLA in a triglyceride or other appropriate form.
5. A topical patch or other topical dosage form in which an appropriate oestrogen dose such as 25 microg was combined with 200-500mg of GLA or DGLA in a triglyceride or ester form which could penetrate the skin without irritation.

## Claims

1. A method of making a medicament for the treatment or prevention of hormone replacement therapy-associated breast pain in peri- or post-menopausal women, wherein an n-6 or n-3 series essential fatty acid is used as an active of said medicament, optionally with a natural or synthetic replacement therapy oestrogen.

2. A method according to claim 1 wherein the essential fatty acid is GLA and/or DGLA optionally with other n-6 and/or n-3 series essential fatty acid(s).

3. A method according to claim 1 wherein the medicament is suited to administration of 1mg to 10g preferably 50mg to 2g and very preferably 100mg to lg of the or each fatty acid daily, and an effective amount of the oestrogen when present in the medicament.

4. A composition of one or more of the essential fatty acids set out in claim 1, 2 or 3 together with an oestrogen as set out therein.

5. A method of treatment or prevention of breast pain arising as set out in claim 1, 2 or 3 wherein an essential fatty acid as set out therein is administered in effective amounts to a woman suffering from or at risk of such pain, separately from or together with a replacement-therapy oestrogen.
